# EUROPEAN PATENT APPLICATION

(11) **EP 1 466 976 A1**
(43) Date of publication of application: **13.10.2004**
(21) Application number: 03700510.5
(22) Date of filing: 09.01.2003
(51) Int. Cl.: C12N 15/12, C12P 21/02, C07K 14/47, C07K 19/00, C12N 1/21

(54) **PROCESS FOR PRODUCING KiSS-1 PEPTIDE**

(30) Priority: 11.01.2002 JP 2002005180
(71) Applicant: Takeda Chemical Industries, Ltd., Osaka-shi, Osaka 541-0045 (JP)
(72) Inventor: YAMADA, Takao, Matsubara-shi, Osaka 580-0003 (JP); TSUJI, Isamu, Mino-shi, Osaka 562-0045 (JP); MISUMI, Yuko, Kawanishi-shi, Hyogo 666-0037 (JP)
(74) Representative: Lewin, John Harvey
(86) International application number: PCT/JP2003/000113
(87) International publication number: WO 2003/060125

(57) **Abstract**

A KiSS-1 Peptide or a salt thereof can be industrially mass-produced by subjecting a fused protein or peptide in which the KiSS-1 peptide is ligated to the N-terminal of a low-molecular peptide having cysteine at its N-terminal to the cleavage reaction of the peptide linkage on the amino group side of said cysteine residue.

## Description

### Technical Field

The present invention relates to a method for producing a KiSS-1 peptide or a salt thereof by preparing a fused protein or polypeptide in which the KiSS-1 peptide is ligated to the N-terminal of a low-molecular peptide having cysteine at its N-terminal, and then subjecting said fused protein or polypeptide to the cleavage reaction of the peptide linkage.

### Background Art

In order to produce peptides using a genetic engineering technology, the peptides expressed in the form of fused proteins is frequently employed since peptides are susceptible to be cleaved intracellularly. Known methods of cleaving the desired peptide from the fused protein include a chemical method using bromocyan (Itakura et al., Science, 198, 1056 (1977)) or an enzymatic method using Factor-Xa (Nagai et al., Methods in Enzymology, 153, 46 (1987)).

Further, as a method of cleaving the peptide linkage in a protein, it is known that acylcysteine bond is cleaved by 2-nitro-5-thiocyanobenzoic acid (Chemistry of Protein II of Lectures on Biochemical Experiment 1, edited by Japanese Biochemical Society and published by Tokyo Kagaku Dozin, pp. 247-250, 1976). However, there has been no disclosure on the excision of the desired peptide from a protein.

WO00/24890 and WO01/75104 disclose a KiSS-1 peptide or a salt thereof, which is used in the present invention.

WO01/44469 discloses a method for producing a KiSS-1 peptide or a salt thereof, which comprises subjecting a fused protein, a peptide or a salt thereof in which the KiSS-1 peptide is ligated to the N-terminal of a protein or peptide having cysteine at its N-terminal, to the cleavage reaction of the peptide linkage on the amino group side of said cysteine residue. The protein or peptide having cysteine at its N-terminal includes interferons, interleukins, various growth factors such as fibroblast growth factor (aFGF, bFGF), (pro)urokinases, lymphotoxin, Tumor Necrosis Factor (TNF), enzyme proteins such as β-galactosidase, storage proteins, streptoavidin, protein A, protein G, Tissue Plasminogen Activator (TPA), or muteins or a part thereof (a fragment).

The prior art method which involves use of bromocyan cannot be applied to the production of methionine-containing peptides, while the method has drawbacks, for example in terms of excision yield.

Therefore, a demand exists for a technology by which the desired protein or peptide may be efficiently excised from the fused protein or polypeptide.

### Disclosure of invention

The present inventors have investigated in detail on a method of efficiently producing a KiSS-1 peptide or a salt thereof, which is a novel physiologically active peptide. As a result, the present inventors have discovered that a KiSS-1 peptide or a salt thereof can be efficiently produced by preparing a fused protein or polypeptide, in which a KiSS-1 peptide is ligated to the N-terminal of a low-molecular peptide having cysteine at its N-terminal, and then subjecting the fused protein or polypeptide to the cleavage reaction of the peptide linkage.

That is, the present invention provides:
(1) A method for producing a KiSS-1 peptide or a salt thereof, which comprises subjecting a fused protein, a peptide or a salt thereof in which the KiSS-1 peptide is ligated to the N-terminal of a low-molecular peptide having cysteine at its N-terminal to the cleavage reaction of the peptide linkage on the amino group side of said cysteine residue;
(2) A method for producing a KiSS-1 peptide or a salt thereof, which comprises cultivating a transformant having a vector comprising a DNA encoding a fused protein or a peptide in which the KiSS-1 peptide is ligated to the N-terminal of a low-molecular peptide having cysteine at its N-terminal to express the fused protein, the peptide or a salt thereof, and subjecting the expressed fused protein, the peptide, or the salt thereof to the cleavage reaction of the peptide linkage on the amino group side of said cysteine residue;
(3) The method according to the above (1) or (2), wherein the C-terminal of the KiSS-1 peptide is an amide;
(4) The method according to the above (1) or (2), wherein the cleavage reaction is an S-cyanylation reaction followed by an ammonolysis or a hydrolysis;
(5) The method according to the above (1) or (2), wherein the KiSS-1 peptide is a peptide comprising an amino acid sequence of SEQ ID NO: 1;
(6) The method according to the above (1) or (2), wherein the KiSS-1 peptide is (i) a peptide having an amino acid sequence of position 40 to position 54 from the N-terminal in the amino acid sequence of SEQ ID NO: 1; (ii) a peptide having an amino acid sequence of position 45 to position 54 from the N-terminal in the amino acid sequence of SEQ ID NO: 1; (iii) a peptide having an amino acid sequence of position 46 to position 54 from the N-terminal in the amino acid sequence of SEQ ID NO: 1; or (iv) a peptide having an amino acid sequence of position 47 to position 54 from the N-terminal in the amino acid sequence of SEQ ID NO: 1;
(7) The method according to the above (1) or (2), wherein the low-molecular peptide having cysteine at the N-terminal is a peptide having cycteine at the N-terminal and consisting of about 10 to about 50 amino acid residues;
(8) The method according to the above (1) or (2), wherein the low-molecular peptide is a partial peptide on the C-terminal side of a precursor protein comprising the KiSS-1 peptide, and has an amino acid sequence starting from an amino acid residue adjacent to the C-terminal amino acid of the KiSS-1 peptide;
(9) The method according to the above (1) or (2), wherein the low-molecular peptide having cysteine at the N-terminal is a peptide that comprises the amino acid sequence of SEQ ID NO: 3 and has a cysteine residue at the N-terminal;
(10) The method according to the above (1) or (2), wherein the low-molecular peptide having cysteine at the N-terminal is a peptide that comprises an amino acid sequence of SEQ ID NO: 3 and has cysteine residue at the N-terminal; the KiSS-1 peptide is a peptide having an amino acid sequence of SEQ ID NO: 1; and the KiSS-1 peptide to be produced is a peptide having an amino acid sequence of SEQ ID NO: 1 with an amide form of the C-terminal;
(11) A fused protein, a peptide, or a salt thereof, in which a KiSS-1 peptide is ligated to the N-terminal of a low-molecular peptide having cysteine at its N-terminal;
(12) The fused protein, the peptide or the salt thereof according to the above (11), comprising an amino acid sequence of SEQ ID NO: 5;
(13) DNA comprising DNA encoding the fused protein or the peptide according to the above (11);
(14) DNA according to the above (13), having (i) a base sequence of SEQ ID NO: 6; or (ii) a base sequence of SEQ ID NO: 7;
(15) A vector comprising the DNA according to the above (13);
(16) A transformant having the vector according to the above (15); and
(17) Escherichia coli MM294 (DE3)/pTC2MetC24-1.3 identified as FERM BP-7823.

Further, the present invention provides:
(18) The method according to the above (2), comprising the following steps (i) to (iv) of:
   (i) producing a DNA encoding a fused protein or a peptide, in which a KiSS-1 peptide is ligated to the N-terminal cysteine of a low-molecular peptide having cysteine at its N-terminal;
   (ii) producing a vector comprising said DNA;
   (iii) cultivating a transformant having said vector to express the fused protein, the peptide or a salt thereof; and
   (iv) subjecting the expressed fused protein, the peptide, or the salt thereof to the cleavage reaction of the peptide linkage on the amino group side of said cysteine residue;
(19) a method for producing a target mature peptide or a salt thereof, which comprises subjecting a fused protein, a peptide or a salt thereof in which the target mature peptide is ligated to the N-terminal of a low-molecular peptide having cysteine at its N-terminal (wherein the low-molecular peptide means a partial peptide on the C-terminal side of a precursor protein of the target mature peptide), to the cleavage reaction of the peptide linkage on the amino group side of said cysteine residue;
(20) A method for producing a target mature peptide or a salt thereof, which comprises cultivating a transformant having a vector comprising DNA encoding a fused protein or a peptide in which the target mature peptide is ligated to the N-terminal of a low-molecular peptide having cysteine at its N-terminal (wherein the low-molecular peptide means a partial peptide on the C-terminal side of a precursor protein of the target mature peptide) to express the fused protein, the peptide, or a salt thereof; and subjecting the expressed fused protein, the peptide, or the salt thereof to the cleavage reaction of the peptide linkage on the amino group side of said cysteine residue;
(21) The method according to the above (19) or (20), wherein the cleavage reaction is an S-cyanylation reaction followed by an ammonolysis or a hydrolysis;
(22) The method according to the above (19) or (20), wherein the target mature peptide is a peptide comprising about 10 to 100 amino acid residues;
(23) The method according to the above (19) or (20), wherein the low-molecular peptide having cysteine at the N-terminal is a peptide consisting of about 10 to about 50 amino acid residues;
(24) A fused protein, a peptide, or a salt thereof, in which a target mature peptide is ligated to the N-terminal of a low-molecular peptide having cysteine at its N-terminal (wherein the low-molecular peptide means a partial peptide on the C-terminal side of a precursor protein of the target mature peptide);
(25) DNA comprising DNA encoding the fused protein or the peptide according to the above (24);
(26) A vector comprising the DNA according to the above (25);
(27) A transformant having the vector according to the above (26); and
(28) The method according to the above (20), comprising the following steps (i) to (iv) of:
   (i) producing a DNA encoding a fused protein or a peptide in which a target mature peptide is ligated to the N-terminal of a low-molecular peptide having cysteine at its N-terminal (wherein the low-molecular peptide means a partial peptide on the C-terminal side of a precursor protein of the target mature peptide);
   (ii) producing a vector comprising said DNA;
   (iii) cultivating a transformant having said vector to express the fused protein, the peptide, or the salt thereof; and
   (iv) subjecting the expressed fused protein, the peptide, or the salt thereof to the cleavage reaction of the peptide linkage on the amino group side of said cysteine residue.

### Brief Description of the Drawings

Fig. 1 shows the DNA fragments used in Example 1;
Fig. 2 shows the schematic diagram of the construction of plasmid pTC2MetC24 obtained in Example 1; and
Fig. 3 shows the schematic diagram of the construction of the plasmid pTC2MetC24 obtained in Example 5.

### Best Mode for Carrying Out the Invention

The KiSS-1 peptide used in the method of the present invention includes, for example, the human KiSS-1 peptide described in WO00/24890, and the mouse or rat KiSS-1 peptide described in WO01/75104.

The human KiSS-1 peptide specifically includes a peptide that contains a sequence of position 47 to position 54 from the N-terminal in the amino acid sequence of SEQ ID NO: 1, and consists of 8 to 54 amino acid residues, etc.

The "peptide that contains a sequence of position 47 to position 54 from the N-terminal in the amino acid sequence of SEQ ID NO: 1, and consists of 8 to 54 amino acid residues" may be selected from any peptides which contain a sequence of position 47 to position 54 from the N-terminal in the amino acid sequence of SEQ ID NO: 1 and consist of 8 to 54 amino acid residues, provided that the peptide activity (for example, the binding activity between the peptide and its receptor, or cell stimulation activity on the cell expressing the receptor caused by the peptide, etc.) is retained to the substantially same extent. Specifically, the following may be used: (i) a peptide having an amino acid sequence of SEQ ID NO: 1; and (ii) a peptide containing a sequence of position 47 to position 54 from the N-terminal in the amino acid sequence SEQ ID NO: 1 at the C-terminal, and consisting of 8 to 15 amino acid residues.

More specifically, the following may be used as the human KiSS-1 peptide: (i) a peptide having an amino acid sequence of SEQ ID NO: 1; (ii) a peptide containing a sequence of position 40 to position 54 from the N-terminal in the amino acid sequence SEQ ID NO: 1; (iii) a peptide containing a sequence of position 45 to position 54 from the N-terminal in the amino acid sequence of SEQ ID NO: 1; (iv) a peptide containing a sequence of position 46 to position 54 from the N-terminal in the amino acid sequence SEQ ID NO: 1; and (v) a peptide containing a sequence of position 47 to position 54 from the N-terminal in the amino acid sequence SEQ ID NO: 1.

As the mouse KiSS-1 peptide (A), for example, (i) a peptide containing a sequence of position 134 to position 141 from the N-terminal in the amino acid sequence of SEQ ID NO: 16 and consisting of 8 to 52 amino acid residues, may be used. Specifically, the following may be used as the mouse KiSS-1 peptide: (i) a peptide containing a sequence of position 90 to position 141 from the N-terminal in the amino acid sequence of SEQ ID NO: 16; (ii) a peptide containing a sequence of position 132 to position 141 from the N-terminal in the amino acid sequence SEQ ID NO: 16; and (iii) a peptide containing a sequence of position 127 to position 141 from the N-terminal in the amino acid sequence of SEQ ID NO: 16.

As the mouse KiSS-1 peptide (B), for example, a peptide containing a sequence of position 138 to position 145 from the N-terminal in the amino acid sequence of SEQ ID NO: 17 and consisting of 8 to 52 amino acid residues, may be used. Specifically, a peptide containing a sequence of position 94 to position 145 from the N-terminal in the amino acid sequence of SEQ ID NO: 17, may be used.

As the rat KiSS-1 peptide, for example, a peptide containing a sequence of position 112 to position 119 from the N-terminal in the amino acid sequence SEQ ID NO: 18 and consisting of 8 to 52 amino acid residues, may be used. Specifically, the following may be used as the rat KiSS-1 peptide: (i) a peptide containing a sequence of position 68 to position 119 from the N-terminal in the amino acid sequence of SEQ ID NO: 18; (ii) a peptide containing a sequence of position 110 to position 119 from the N-terminal in the amino acid sequence of SEQ ID NO: 18; and (iii) a peptide containing a sequence of position 105 to position 119 from the N-terminal in the amino acid sequence of SEQ ID NO: 18.

The above-mentioned KiSS-1 peptides have a ligand activity to the receptor protein, OT7T175 described in WO00/24890 or WO01/75104.

The peptides of the present specification are designated by the conventional way of describing peptides. That is, the left end is the N-terminal (amino terminal) and the right end is the C-terminal (carboxyl terminal). The C-terminal of the peptide represented by SEQ ID NO: 1 may be amide (-CONH₂), a carboxyl group (-COOH), a carboxylate (-COO-), an alkylamide (-CONHR), or an ester (-COOR). R of the ester or alkylamide includes, for example, a C₁₋₆ alkyl group such as methyl, ethyl, n-propyl, isopropyl, and n-butyl; a C₃₋₈ cycloalkyl group such as cyclopentyl and cyclohexyl; a C₆₋₁₂ aryl group such as phenyl and α-naphthyl; a phenyl-C₁₋₂ alkyl such as benzyl, phenethyl and benzhydryl; or a C₇₋₁₄ aralkyl group such as an α-naphthyl-C₁₋₂ alkyl group, e.g., α-naphthylmethyl; and the like. In addition, a pivaloyloxymethyl group or the like which is used widely as an ester for oral administration may also be used.

Further, the KiSS-1 peptide includes peptides wherein the amino group of N-terminal methionine residue of the above peptide is protected with a protecting group (e.g., C₁₋₆ acyl group such as C₂₋₆ alkanoyl group, e.g., formyl group, acetyl group, etc.); those wherein the N-terminal side is cleaved in vivo and the glutamyl group formed is pyroglutaminated; and those wherein a substituent (e.g., -OH, -SH, -COOH, amino group, imidazole group, indole group, guanidino group, etc.) on the side chains of an amino acid in the molecule of the peptide is protected with an appropriate protecting group (e.g., C₁₋₆ acyl group such as C₂₋₆ alkanoyl group, e.g., formyl group, acetyl group, etc.), or conjugated peptides such as glycopeptides with sugar chains attached thereto.

Salts of the KiSS-1 peptide of the present invention may be ones with physiologically acceptable bases (for example, alkali metals, etc.) or acids (organic acids or inorganic acids), preferably physiologically acceptable acid addition salts thereof. Examples of such salts include salts with inorganic acids (e.g., hydrochloric acid, phosphoric acid, hydrobromic acid, and sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

In the method of the present invention, the proteins or peptides having cysteine at its N-terminal are not specified. If there is no cysteine at the N-terminal, a protein or a peptide may have cysteine at its N-terminal according to a publicly known method.

The "low-molecular peptide" in the low-molecular peptide having cysteine at its N-terminal has, for example, about 10 to 50, preferably about 20 to 40, more preferably about 20 to 30 amino acid residues. Among others, (i) a partial peptide of the human KiSS-1 peptide precursor having the amino acid sequence of SEQ ID NO: 15 (for example, J. Natl. Cancer Inst., 88, 1731, 1996; WO98/39448), (ii) a partial peptide of the mouse KiSS-1 peptide precursor (A) having the amino acid sequence of SEQ ID NO: 16 (WO01/75104), (iii) a partial peptide of the mouse KiSS-1 peptide precursor (B) having the amino acid sequence of SEQ ID NO: 17 (WO01/75104), (iv) a partial peptide of the rat KiSS-1 peptide precursor having the amino acid sequence of SEQ ID NO: 18 (WO01/75104) and the like may be used. Among others, a partial peptide on the C-terminal side of such KiSS-1 peptide precursors is preferred. More preferably, the low-molecular peptide is a partial peptide on the C-terminal side of the precursor protein containing the KiSS-1 peptide, and has an amino acid sequence starting from the amino acid residues adjacent to the C-terminal amino acid of the KiSS-1 peptide, etc.

More specifically, the following may be used as the low-molecular peptide:
(1) When the KiSS-1 peptide is the human KiSS-1 peptide, a partial peptide on the C-terminal side of the human KiSS-1 peptide precursor having the amino acid sequence of SEQ ID NO:3, etc.;
(2) When the KiSS-1 peptide is the mouse KiSS-1 peptide (A), a partial peptide on the C-terminal side of the mouse KiSS-1 peptide precursor containing a sequence of position 142 to position 152 from the N-terminal in the amino acid sequence of SEQ ID NO: 16;
(3) When the KiSS-1 peptide is the mouse KiSS-1 peptide (B), a partial peptide on the C-terminal side of the mouse KiSS-1 peptide precursor containing a sequence of position 146 to position 156 from the N-terminal in the amino acid sequence of SEQ ID NO: 17; and
(4) When the KiSS-1 peptide is the rat KiSS-1 peptide, a partial peptide on the C-terminal side of the rat KiSS-1 peptide precursor containing a sequence of position 120 to position 130 from the N-terminal in the amino acid sequence of SEQ ID NO: 18.

In the method of the present invention, cysteine is ligated to the N-terminal of such low-molecular peptides.

The DNA encoding the fused protein (including the fused peptides) used for the method of the present invention may be constructed by (1) chemically synthesizing the entire base sequence, or (2) placing a base sequence encoding cysteine on the N-terminal side of a base sequence encoding a low-molecular peptide and placing a base sequence encoding a KiSS-1 peptide on the N-terminal side. Also, when it is desired to obtain a fragment of said peptide, the DNA may be constructed by (3) substituting the amino acid residue immediately after the desired fragment with cysteine by, for example, site-directed mutagenesis or other technique.

In the case of the above (1), the desired DNA can be prepared by ligation using T4 DNA ligase after synthesis of the entire sequence at once if it is short or in separate steps if it is long, by the publicly known phosphoamidide method, phosphotriester method, diester method or hydrogen phosphonate method.

In the case of the above (2), the desired DNA can be obtained as follows: The DNA encoding the C-terminal protein is obtained by cleavage from chromosome or cDNA with the appropriate restriction enzymes and subsequent ligation with a vector, or by obtaining a cDNA, which is then cleaved with restriction enzymes so that cysteine is present at its N-terminal or modified to have cysteine at its N-terminal by ligating a synthetic DNA to the 5'-terminal of the entire protein or partial gene thereof. The 5'-terminal is ligated to a DNA encoding the desired protein (which DNA may be chemically synthesized or may be cloned from an organism).

Examples of the thus obtained DNA encoding the fused protein include the DNA containing a base sequence (SEQ ID NO: 6 or 7) represented by the following formula: 
GGTACTTCTCTGTCTCCGCCGCCGGAATCTTCTGGTTCTCGTCAGCAGCC
GGGTCTGTCTGCTCCGCACTCTCGTCAGATCCCGGCTCCGCAGGGTGCT
GTTCTGGTTCAGCGTGAAAAAGACCTGCCGAACTACAACTGGAACTCTTT
CGGTCTGCGTTTC(SEQ ID NO: 2)-TGC or TGT-R (I)
 [wherein R represents the base sequence represented by SEQ ID NO: 4].

The above formula (I) shows that the base sequence represented by R (SEQ ID NO: 4) is ligated to the DNA base sequence (SEQ ID NO: 2) encoding a peptide comprising a human KiSS-1 peptide through the base sequence encoding cysteine (TGC or TGT).

The DNA encoding the human KiSS-1 peptide may also be produced according to any publicly known method using DNA represented by the formula (I), DNA encoding the human KiSS-1 peptide precursor containing the amino acid sequence of SEQ ID NO: 15, or modified DNA thereof (for example, J. Natl. Cancer Inst., 88, 1731, 1996; WO98/39448).

The DNA encoding the mouse KiSS-1 peptide may be produced according to any publicly known method using DNA (SEQ ID NO: 19) encoding a mouse KiSS-1 peptide precursor (A) having the amino acid sequence of SEQ ID NO: 16, or modified DNA thereof (WO01/75104), or DNA (SEQ ID NO: 20) encoding a mouse KiSS-1 peptide precursor (B) having the amino acid sequence of SEQ ID NO: 17, or a modified DNA thereof (WO01/75104).

The DNA encoding the rat KiSS-1 peptide may be produced according to any publicly known method using DNA (SEQ ID NO: 21) encoding a rat KiSS-1 peptide precursor having the amino acid sequence of SEQ ID NO: 18, or a modified DNA thereof (WO01/75104).

A DNA (plasmid) which has ATG at its 5'-terminal, a region encoding the fused protein downstream thereof and a translation termination codon further downstream can be produced by chemical synthesis or by processing known cDNA of said protein produced by genetic engineering or by processing the chromosome-derived DNA of said protein.

In the present invention, the DNA encoding a fused protein or a peptide in which a KiSS-1 peptide is ligated to the N-terminal of the low-molecular peptide having cysteine at its N-terminal, can be modified to DNA encoding the desired mutein by using the conventional DNA technology, for example, site-directed mutagenesis.

Site-directed mutagenesis is well-known, and it is described in Genetic Engineering, Lather, R. F. and Lecoq, J. P., Academic Press, pp. 31 to 50 (1983). Mutagenesis directed to oligonucleotide is described in Genetic Engineering: Principles and Methods, Smith, M. and Gillam, S., Plenum Press, Vol. 3, pp. 1 to 32 (1981).

Examples of the plasmid used as a vector to produce a plasmid carrying DNA having a region coded for said fused protein include Escherichia coli-derived plasmids such as pBR322 [Gene, 2, 95 (1977)], pBR313 [Gene, 2, 75 (1977)], pBR324, pBR325 [Gene, 4, 124 (1978)], pBR327, pBR328 [Gene, 9, 287 (1980)], pBR329 [Gene, 17, 79 (1982)], pKY2289 [Gene, 3, 1 (1978)], pKY2700 [Seikagaku, 52, 770 (1980)], pACYC177, pACYC184 [Journal of Bacteriology, 134, 1141 (1978)], pRK248, pRK646, pDF [Methods in Enzymology, 68, 268 (1979)], pUC18, pUC19 [Janisch-Perror et al., Gene, 33, 103 (1985)], or the like. Also useful are λgt vectors using λ phage such as λgt-λC [Proc. Nat. Acad. Sci., U.S.A., 71, 4579 (1974)], λgt-λB [Proc. Nat. Acad. Sci., U.S.A., 72, 3461 (1975)], λDam [Gene, 1, 255 (1977)], Charon vector [Science, 196, 161 (1977); Journal of Virology, 29, 555 (1979)] and mp vectors using a filamentous phage such as mp18 and mp19 (Janisch-Perror et al., Gene, 33, 103 (1985)].

The DNA preferably has a promoter upstream of ATG. Any promoter may be used as long as it is suitable for the host in producing a transformant.

Examples of such promoters include the trp promoter, lac promoter, rec A promoter, λPL promoter, lpp promoter and T7 promoter for Escherichia coli, SPO1 promoter, SPO2 promoter and penP promoter for Bacillus subtilis, PHO5 promoter, PGK promoter, GAP promoter and ADH promoter for Saccharomyces cerevisiae and the SV40-derived promoter for animal cells. The SD (Shine and Dalgarno) sequence may be inserted downstream of the promoter as necessary.

When using the T7 promoter system, the promoter may be any of the 17 promoters found on the T7 DNA [J. L. Oakley et al., Proc. Natl. Acad. Sci., U.S.A., 74, 4266-4270 (1977); M. D. Rosa, Cell, 16, 815-825 (1979); N. Panayotatos et al., Nature, 280, 35 (1979); J. J. Dunn et al., J. Mol. Biol., 166, 477-535 (1983)]. The φ10 promoter [A. H. Rosenberg et al., Gene, 56, 125-135 (1987)] is most preferable.

Any transcription terminator can be used, as long as it functions in Escherichia coil systems, but the Tφ terminator [F. W. Studier et al., J. Mol. Biol., 189, 113-130 (1986)] is preferred.

The T7 RNA polymerase gene is exemplified by the T7 gene [F. W. Studier et al., J. Mol. Biol., 189, 113-130 (1986)].

The vector is preferably constructed by inserting the T7 promoter and T7 terminator into the above-mentioned vector. Examples of such vectors include pET-1, pET-2, pET-3, pET-4 and pET-5 [A. H. Rosenberg, Gene, 56, 125-135 (1987)], and pTB960-2 (EP-A-499990)), but pTB960-2 is preferably used.

The transformant of the present invention can be produced by transforming a host with an expression plasmid obtained as described above using a publicly known method [e.g., Cohen S. N. et al., Proceedings of National Academy of Science, U.S.A., 69, 2110 (1972)].

Examples of the microorganism host to be transformed include bacteria belonging to the genus Escherichia, bacteria belonging to the genus Bacillus, yeasts, and animal cells.

Examples of the bacteria belonging to the genus Escherichia include Escherichia coli (E. coli), specifically Escherichia coli K12DH1 [Proceedings of National Academy of Science, U.S.A., 60, 160 (1968)], JM-103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], N4830 [Cell, 25, 713 (1981)], K-12MM294 [Proceedings of National Academy of Science, U.S.A., 73, 4174 (1976)] and BL21.

Examples of the bacteria belonging to the genus Bacillus include Bacillus subtilis, specifically Bacillus subtilis MI114 [Gene, 24, 255 (1983)] and 207-21 [Journal of Biochemistry, 95, 87 (1984)].

Examples of the yeasts include Saccharomyces cerevisiae, specifically Saccharomyces cerevisiae AH22 [Proceedings of National Academy of Science, U.S.A., 75, 1929 (1978)], XSB52-23C [Proceedings of National Academy of Science, U.S.A., 77, 2173 (1980)], BH-641A (ATCC 28339), 20B-12 [Genetics, 85, 23 (1976)] and GM3C-2 (Proceedings of National Academy of Science, U.S.A., 78, 2258 (1981).

Examples of the animal cells include simian cells COS-7 [Cell, 23, 175 (1981)], Vero [Japanese Journal of Clinical Medicine, 21, 1209 (1963)], Chinese hamster cells CHO [Journal of Experimental Medicine, 108, 945 (1985)], mouse L cells [Journal of National Cancer Institute, 4, 165 (1943)], human FL cells [Proceedings of the Society for Experimental Biology and Medicine, 94, 532 (1957)] and hamster C cells.

When using a T7 promoter system, any host can be used to obtain the desired transformant, as long as it is an Escherichia coli strain capable of incorporating the T7 RNA polymerase gene (T7 gene 1) [F. W. Studier et al., J. Mol. Biol., 189, 113-130 (1986)]. Examples of such strains are MM294, DH-1, C600, JM109, BL21 or an Escherichia coli strain with another plasmid with the T7 RNA polymerase gene (T7 gene 1). It is preferable to use the MM294 strain or BL21 strain resulting from lysogenization of a λ phage incorporating the T7 gene 1. In this case, the lac promoter is used as promoter for the T7 gene 1, which induces expression in the presence of isopropyl-1-thio-β-D-galactopyranoside (which is abbreviated to IPTG).

The transformation of Escherichia bacteria can be carried out using a known method such as that disclosed in Proceedings of National Academy of Science, U.S.A., 69, 2110 (1972), and Gene, vol. 17, 107 (1982).

The transformation of the host of the genus Bacillus, can be carried out using a known method such as that disclosed in Molecular & General Genetics, 168, 111 (1979).

The transformation of the host of yeast, can be carried out using a known method such as that disclosed in Proc. Natl. Acad. Sci. USA, 75, 1929 (1978).

The transformation of the host of an animal cell can be carried out using a known method such as that disclosed in Virology, 52, 456 (1973).

The fused protein can be produced by cultivating the above-mentioned transformant in a cultured medium and then harvesting the produced fused protein.

It is desirable that the pH of medium be about 6 to 8.

An example of a medium for cultivating the bacteria of the genus Escherichia is the M9 medium containing glucose and casamino acids [Miller, Journal of Experiments in Molecular Genetics, 431-433, Cold Spring Harbor Laboratory, New York (1972)]. A chemical substance such as 3β-indolylacrylic acid and isopropyl-β-D-thiogalactopyranoside (IPTG) may be added thereto, when it is necessary to increase promoter efficiency.

When the host is a bacterium of the genus Escherichia, cultivation is normally carried out at about 15 to 43°C for about 3 to 24 hours, and aeration and/or agitation may also be performed, if necessary.

When the host is a bacterium of the genus Bacillus, cultivation is normally carried out at about 30 to 40°C for about 6 to 24 hours, and aeration and/or agitation may also be performed, if necessary.

As a medium for the cultivation of transformants whose host is a yeast, Burkholder's minimum medium may be used [Bostian, K. L. et al., Proc. Natl. Acad. Sci. USA, 77, 4505 (1980)]. It is preferable that the pH of the medium be adjusted to about 5 to 8. Cultivation is normally carried out at about 20 to 35°C for about 24 to 72 hours, and aeration and/or agitation may also be performed, if necessary.

Media for the cultivation of transformants whose host is an animal cell include MEM media containing about 0.2 to 20%, preferably about 5 to 20% fetal bovine serum [Science, 122, 501 (1952)], DMEM medium [Virology, 8, 396 (1959)], RPMI1640 medium [Journal of the American Medical Association, 199, 519 (1967)] and 199 medium [Proceeding of the Society for the Biological Medicine, 73, 1 (1950)]. It is preferable that pH be about 6 to 8. Cultivation is normally carried out at about 30 to 40°C for about 15 to 60 hours, and aeration and/or agitation may be performed, if necessary.

The fused protein can be produced by cultivating the above-mentioned transformant, to produce and accumulate the fused protein in a cultured medium and then harvesting it.

Examples of media include the M9 medium containing glucose and casamino acids [Miller, Journal of Experiments in Molecular Genetics, 431-433, Cold Spring Harbor Laboratory, New York (1972)] and 2 × YT medium [Messing, Methods in Enzymology, 101, 20 (1983)] and LB medium.

Cultivation is normally carried out at about 15 to 43°C for about 3 to 24 hours, and aeration and/or agitation may also be performed, if necessary.

When using a recombinant vector having both a λcIts repressor and an expression vector carrying a λPL-promoter, it is preferable to carry out cultivation of the transformant at a temperature between about 15 and 36°C, preferably about 30 and 36°C and inactivate the λcIts repressor at a temperature between about 37 and 42°C. Also, to increase recA promoter efficiency, i.e., to lower the recA gene expression suppressive function, a drug such as mitomycin C or nalidixic acid may be added, ultraviolet irradiation may be employed, or pH of the medium may be changed to alkali, if necessary.

When using a T7 promoter system, (1) IPTG is added to express the T7 gene (RNA polymerase gene) ligated downstream from the lac promoter; or (2) the temperature of the culture medium may be elevated in expressing the T7 gene (RNA polymerase gene) ligated downstream of the λP_{L}-promoter to specifically activate the T7 promoter via the resulting T7 phage RNA polymerase 1.

After cultivation, cells are collected by a known method and suspended in a buffer, after which they are disrupted by protein denaturant treatment, ultrasonic treatment, enzymatic treatment using lysozyme, glass bead treatment, French press treatment, freeze-thawing or other process, followed by centrifugation or other known methods, to yield a supernatant.

From the supernatant thus obtained, the fused protein can be isolated in accordance with known methods of protein purification. For example, gel filtration, ion exchange chromatography, adsorption chromatography, high performance liquid chromatography, affinity chromatography, hydrophobic chromatography and electrophoresis can be used in appropriate combination. The fused protein may be subjected to the subsequent reaction process without purification or in a partially purified state.

The fused protein or peptide thus obtained is then subjected to a reaction for cleaving the peptide linkage on the amino group side of the cysteine residue. The cleavage reaction may be, for example, performed by S-cyanylation reaction followed by hydrolysis. If an amide form of KiSS-1 peptide or a salt thereof is desired as the final product, the cleavage reaction may be performed by the S-cyanylation followed by ammonolysis. The S-cyanylation reaction is carried out by reacting an S-cyanylation reagent with the starting compounds.

Examples of S-cyanylation reagents include 2-nitro-5-thiocyanobenzoic acid (NTCB), 1-cyano-4-dimethylaminopyridium salt (DMAP-CN) and CN⁻ ion. The amount of S-cyanylation reagent is about 2 to 50 times, preferably about 5 to 10 times the total amount of all thiol groups.

Reaction temperature may be set at any level in the range from about 0 to 80°C., preferably between about 0 and 50°C. Any buffer can be used as a solvent, as long as it does not react with the S-cyanylation reagent. Examples of such buffers include Tris-HCl buffer, Tris-acetate buffer, phosphate buffer and borate buffer. An organic solvent may be present, as long as it does not react with the S-cyanylation reagent.

The reaction may be normally carried out at a pH of 1 to 12. Particularly when using NTCB, a pH range of from 7 to 10 is preferred. When using DMAP-CN, a pH range of from 2 to 7 is preferred, in order to avoid S-S exchange reaction. The reaction mixture may contain a denaturant such as guanidine hydrochloride.

Reaction for cleaving by hydrolysis or ammonolysis can be achieved, for example, by alkali treatment.

The alkali treatment is carried out by adjusting a pH of the aqueous solution containing the starting compound to 7 to 14.

The pH can be adjusted by adding an appropriate amount of a solution of ammonia, sodium hydroxide, amino compound, trizma base (tris[hydroxymethyl]-aminomethane), disodium phosphate, potassium hydroxide or barium hydroxide, to the aqueous solution containing the starting compound. In particular, ammonia, etc. is preferable.

The concentration of said solution are, for ammonia or amino compound about 0.01 to 15 N, preferably about 0.1 N to 3 N; for sodium hydroxide about 0.01 to 2 N, preferably about 0.05 N to 1 N; for trizma base about 1 mM to 1 M, preferably about 20 mM to 200 mM; for disodium phosphate about 1 mM to 1 M, preferably about 10 mM to 100 mM; for potassium hydroxide about 0.01 to 4 N, preferably about 0.1 to 2 N. The reaction temperature may be between about -20 to 80°C, preferably between about -10 to 50°C.

Reaction times are preferably as follows: for S-cyanylation about 10 to 60 minutes, preferably about 15 to 30 minutes; for hydrolysis about 5 minutes to 100 hours, preferably about 10 minutes to 15 hours; for ammonolysis about 5 minutes to 24 hours, preferably about 10 to 180 minutes.

Examples of the amino compound include a compound represented by the formula R¹-(NR²)-H wherein R¹ and R² may be the same or different and represent (i) a hydrogen atom; (ii) a C₁₋₂₀ alkyl group, a C₃₋₈cycloalkyl group, a C₆₋₁₄ aryl group, or a C₆₋₁₄ aryl-C₁₋₃ alkyl group (these may have no substituent, or may have 1 to 3 amino groups, hydroxyl groups and the like, on a carbon atom); (iii) an optionally substituted amino group; and (iv) a hydroxyl group or a C₁₋₆ alkoxy group.

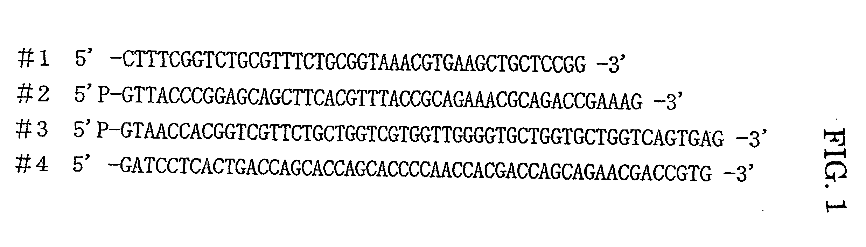

The reaction shown in Fig. 1 is considered to occur by S-cyanylation, and ammonolysis or hydrolysis as described above.

As described above, the C-terminal of the KiSS-1 peptide obtainable by a production method of the present invention may be amide (-CONH₂), carboxyl group (-COOH), carboxylate (-COO-), alkylamide (-CONHR), or ester (-COOR). Amide, carboxyl group or alkylamide is preferable, and in particular, amide or alkylamide is suitable. Specifically, the C-terminal of the KiSS-1 peptide obtainable by the producing method of the present invention may be -CO-X shown in Fig. 1, wherein X represents R¹-(NR²)- (R¹ and R² are the same as defined above) or OH.

Examples of the C₁₋₂₀ alkyl group include methyl, ethyl, propyl, isopropyl, butyl, sec-butyl, pentyl, isopentyl, neopentyl, 1-ethylpentyl, hexyl, isohexyl, heptyl, octyl, nonanyl, decanyl, undecanyl, dodecanyl, tetradecanyl, pentadecanyl, hexadecanyl, heptadecanyl, octadecanyl, nonadecanyl, and eicosanyl.

Examples of the C₃₋₈ cycloalkyl group include cyclopropyl, cyclobutyl, cyclopentyl, cyclohexyl, cycloheptyl, and cyclooctyl.

Examples of the C₆₋₁₄ aryl group include phenyl, naphthyl, anthryl, phenanthryl, and acenaphthylenyl.

Examples of the C₆₋₁₄ aryl-C₁₋₃ alkyl group include benzyl, phenethyl, 3-phenylpropyl, (1-naphthyl)methyl, and (2-naphthyl)methyl.

Examples of the C₁₋₆ alkoxy group include methoxy, ethoxy, propoxy, butoxy, pentyloxy, and hexyloxy.

As the substituents of the optionally substituted amino group in the above (iii), an amino acid and a peptide comprising 2 to 10 amino acids are exemplified.

Examples of the amino acid include L- or D-isomer of Ala Arg, Asp, Asn, Glu, Gln, Gly, His, Ile, Met, Leu, Phe, Pro, Ser, Thr, Trp, Tyr, and Val.

Examples of the peptide include HD-Leu-Leu-Arg-Pro-NH--C₂H₅, and H-Val-Ala-Leu-D-Ala-Ala-Pro-Leu-Ala-Pro-Arg-OH.

Among others, it is preferred that R² be a hydrogen atom, and R¹ be a hydrogen atom or a C₁₋₂₀ alkyl group.

When using ammonia or an amino compound in the ammonolysis reaction, the corresponding amide form is obtained.

The desired peptide obtained through cleavage may be isolated in accordance with known methods of protein purification. For example, gel filtration, ion exchange chromatography, high performance liquid chromatography, affinity chromatography, hydrophobic chromatography, thin layer chromatography and electrophoresis can be used in appropriate combination.

The KiSS-1 peptide or a salt thereof, thus obtained may also be isolated and purified from the reaction solution by a well-known purification technique, for example, extraction, salting out, distribution, recrystallization, or chromatography. Preferable examples of the purification technique include ion-exchange chromatography through, for example, SP-Sepharose (Pharmacia Biotech, Co., Ltd.), DEAE-5PW (Tosoh Corporation), or SP-5PW (Tosoh Corporation), etc.

The KiSS-1 peptide or the salt thereof obtained can be powdered by lyophilization, if necessary. In lyophilization, a stabilizer such as sorbitol, mannitol, dextrose, maltose, trehalose or glycerol may be added.

The KiSS-1 peptide or a salt thereof produced by the method of the present invention can be mixed with sterile water, human serum albumin (HSA), physiological saline and other known physiologically acceptable carriers, and can be administered parenterally or topically to a mammalian (e.g. human). It can be administered parenterally by intravenous, intramuscular or other means at a daily dose of about 0.01 mg to 50 mg, preferably about 0.1 mg to 10 mg per one person.

Preparations containing the KiSS-1 peptide or a salt thereof produced by the method of the present invention may contain salts, diluents, adjuvants and other carriers, buffers, binders, surfactants, preservatives and other physiologically acceptable active ingredients. Parenteral preparations are supplied as sterile water solutions, ampules containing a suspension in a physiologically acceptable solvent or ampules containing a sterile powder normally obtained by lyophilizing a peptide solution which can be freshly prepared in dilution with a physiologically acceptable diluent for each use.

The KiSS-1 peptide or a salt thereof produced by the method of the present invention has an activity of inhibiting cancer metastasis, and therefore, is useful as a prophylactic or therapeutic drug for all kinds of cancers (for example, lung cancer, stomach cancer, liver cancer, pancreatic cancer, colorectal cancer, rectal cancer, colon cancer, prostate cancer, ovarian cancer, uterine cancer, or breast cancer, etc.).

In addition, the KiSS-1 peptide or a salt thereof has an activity of controlling placental function, and therefore, is useful as a prophylactic or therapeutic drug for choriocarcinoma, vesicular mole, invasive mole, miscarriage, fetal maldevelopment, saccharometabolic disorder, lipid metabolic disorder, or induction of delivery.

Also, in the method of the present invention, the desired mature peptide can be produced in a similar manner, by substituting KiSS-1 peptide with the other desired mature peptide, and further substituting a low-molecular peptide having cysteine at its N-terminal with the corresponding partial peptide on the C-terminal side of the precursor protein of the desired mature peptide.

Examples of the other desired mature peptide include a peptide having amino acid residues of about 10 to 200, preferably about 10 to 100, etc.

Examples of the partial peptide on the C-terminal side of the precursor protein of the desired mature peptide include a partial peptide on the C-terminal side of a precursor protein containing the desired mature peptide, having an amino acid sequence starting from an amino acid residue adjacent to the C-terminal amino acid residue of the desired mature peptide, etc.

In addition, examples of the partial peptide on the C-terminal side of the precursor protein of the desired mature peptide include a partial peptide having about 10 to 50, preferably about 20 to 40, more preferably about 20 to 30 amino acid residues on the C-terminal side of a precursor protein containing the desired mature peptide.

Abbreviations for amino acids, peptides, protective groups, active groups and other materials used in the present specification and attached drawings are based on abbreviations specified by the IUPAC-IUB (Commission on Biochemical Nomenclature) or abbreviations in common use in relevant fields, examples of which are given below. In addition, for the amino acids that may have the optical isomers, L-form is presented unless otherwise indicated.
DNA: Deoxyribonucleic acid
A : Adenine
T : Thymine
G : Guanine
C : Cytosine
RNA: Ribonucleic acid
EDTA: Ethylenediaminetetraacetic acid
Gly : Glycine
Ala : Alanine
Val : Valine
Leu : Leucine
Ile : Isoleucine
Ser : Serine
Thr : Threonine
Met : Methionine
Glu : Glutamic acid
Asp : Aspartic acid
Lys : Lysine
Arg : Arginine
His : Histidine
Phe : Phenylalanine
Tyr : Tyrosine
Trp : Tryptophan
Pro : Proline
Asn : Asparagine
Gln : Glutamine
Cys : Cysteine
Asx : Asparagine or aspartic acid
Glx : Glutamine or glutamic acid
ATP : Adenosine triphosphate

The sequence identification numbers in the sequence listing of the specification indicates the following sequence, respectively.

### [SEQ ID NO: 1]

This sequence shows the amino acid sequence of the human KiSS-1 peptide.

### [SEQ ID NO: 2]

This sequence shows the base sequence of the DNA encoding the human KiSS-1 peptide.

### [SEQ ID NO: 3]

This sequence shows the amino acid sequence of the low-molecular peptide.

### [SEQ ID NO: 4]

This sequence shows the base sequence of the DNA encoding the low-molecular peptide.

### [SEQ ID NO: 5]

This sequence shows the amino acid sequence of the fused protein.

### [SEQ ID NO: 6]

This sequence shows the base sequence 1 of the DNA fragment encoding the fused protein represented by formula (I).

### [SEQ ID NO: 7]

This sequence shows the base sequence 2 of the DNA fragment encoding the fused protein represented by formula (I).

### [SEQ ID NO: 8]

This sequence shows the base sequence of an oligomer used for preparing the structural gene of the KiSS-1 peptide in Example 1.

### [SEQ ID NO: 9]

This sequence shows the base sequence of the oligomer used for preparing the structural gene of the KiSS-1 peptide in Example 1.

### [SEQ ID NO: 10]

This sequence shows the base sequence of the oligomer used for preparing the structural gene of the KiSS-1 peptide in Example 1.

### [SEQ ID NO: 11]

This sequence shows the base sequence of the oligomer used for preparing the structural gene of the KiSS-1 peptide in Example 1.

### [SEQ ID NO: 12]

This sequence shows the base sequence of the DNA fragment obtained in Example 1.

### [SEQ ID NO: 13]

This sequence shows the base sequence of the DNA fragment obtained in Example 1.

### [SEQ ID NO: 14]

This sequence shows the base sequence of the DNA fragment obtained in Example 1.

### [SEQ ID NO: 15]

This sequence shows the amino acid sequence of the KiSS-1 peptide precursor.

### [SEQ ID NO: 16]

This sequence shows the amino acid sequence of the mouse KiSS-1 peptide precursor (A).

### [SEQ ID NO: 17]

This sequence shows the amino acid sequence of the mouse KiSS-1 peptide precursor (B).

### [SEQ ID NO: 18]

This sequence shows the amino acid sequence of the rat KiSS-1 peptide.

### [SEQ ID NO: 19]

This sequence shows the base sequence of the DNA encoding the mouse KiSS-1 peptide precursor (A).

### [SEQ ID NO: 20]

This sequence shows the base sequence of the DNA encoding the mouse KiSS-1 peptide precursor (B).

### [SEQ ID NO: 21]

This sequence shows the base sequence of the DNA encoding the rat KiSS-1 peptide.

The transformant Escherichia coli MM294 (DE3)/pTC2MetC24-1.3 obtained in Example 1 below has been at the International Patent Organism Depositary (IPOD) of the National Institute of the Advanced Industrial Science and Technology (AIST) Tsukuba Central, 6, 1-1-1 Higashi, Tsukuba, Ibaraki, Japan (Zip code: 305-8566) under the Accession Number FERM BP-7823 since December 10, 2001 and has been deposited at the Institute for Fermentation, Osaka (IFO), Japan under the accession number IFO 16717 since October 24, 2001.

### EXAMPLES

The present invention will be described in more detail below with reference to the following examples, which are merely illustrative, and not intended to limit the present invention.

### Example 1

### (1) Production of DNA encoding a KiSS-1 peptide to which 24 amino acids are added at its C-terminal

### (a) Synthesis of DNA Fragments

The gene of the KiSS-1 peptide to which 24 amino acid residues are added to its C-terminal was prepared using four DNA fragments shown in Fig. 1 (#1, #2; 5'-terminal phosphorylated, #3; 5'-terminal phosphorylated, and #4: products of Kikotech).

### (b) Ligation of the DNA Fragments for adding at the C-terminal

The DNA fragments #1 to #4 obtained in the above (a) were combined to a total volume of 40 µl. This mixture solution was maintained at 65°C for 10 minutes and then gradually cooled to room temperature to effect annealing. The ligation reaction was conducted using T4 DNA Ligase (Takara Shuzo) to 10 µl of the annealing solution. Thus, 2 µl of 10 × concentration of Ligation buffer and 1 µl of T4 DNA Ligase (350 units) were added to 10 µl of the annealing solution, and after thorough mixing, the ligation reaction was carried out at 16°C for 17 hours. Then, heat treatment was conducted at 65°C for 5 minutes. The DNA fragments thus obtained was phosphorylated using T4 polynucleotide kinase (Takara Shuzo). Then, 96 bp DNA fragment (SEQ ID NO: 12) was extracted by 1.8% low melting point agarose gel electrophoresis using an ELUTIP Minicolumn (Schleicher and Schuell), and dissolved in 20 µl of TE buffer, which was used in the next (c) step.

### (c) Ligation of KiSS-1 gene and the DNA Fragments for adding at its C-terminal.

pTFC-KiSS-1, an expression. vector for KiSS-1 peptide, was digested with NdeI (Takara Shuzo) and XmnI (NEB) at 37°C for 2 hours, and 149 bp DNA fragment (SEQ ID NO: 13) was extracted by 1.5% low melting point agarose gel electrophoresis using an ELUTIP Minicolumn (Schleicher and Schuell), and dissolved in 20 µl of TE buffer solution. This DNA fragment and the DNA fragment obtained in the above (b) were subjected to ligation. To 20 µl of 149 bp DNA solution and 10 µl of 96 bp DNA solution were added 3.5 µl of 10 × concentration of Ligation buffer and 1.5 µl of T4 DNA Ligase (525 units), and after thorough mixing, the ligation reaction was conducted at 16°C for 17 hours. After conducting ligation, heat treatment was carried out at 65°C for 5 minutes. After conducting digestion with NdeI and BamHI for 1 hour using this ligation solution in 350 µl of a total volume, a DNA fragment(SEQ ID NO: 14) was recovered by ethanol precipitation. This was used in the next (d) step.

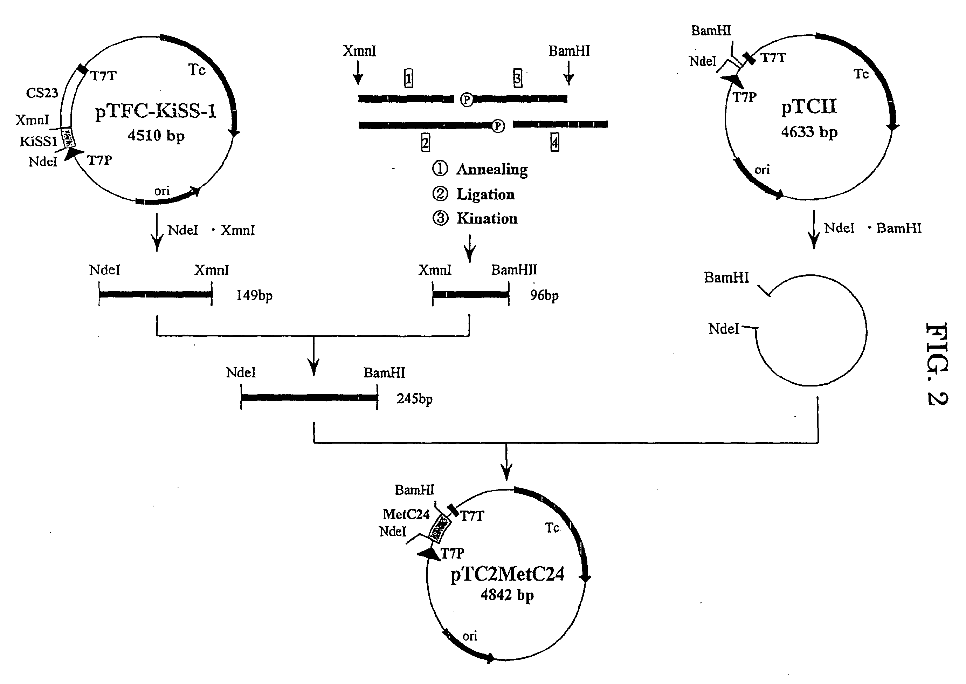

### (d) Construction of the expression vector for KiSS-1 peptide to which 24 amino acids are added at its C-terminal (Fig. 2).

The expression vector pTCII was digested with NdeI and BamHI (Takara Shuzo) at 37°C for 1 hour. A 4.6 kb DNA fragment was extracted by 1% agarose gel electrophoresis using QIAquick Gel Extraction Kit (Qiagen), and dissolved in 25 µl of TE buffer. This NdeI-BamHI fragment of pTCII and the DNA fragment obtained in the above (c) were subjected to ligation reaction using T4 DNA Ligase (Takara Shuzo).

Competent cells of Escherichia coli JM109 (Takara Shuzo) were transformed using 10 µl of this reaction solution, then sowed onto LB agar medium containing 10 µg/ml of tetracycline and cultured overnight at 37°C. A tetracycline-resistant colony thus formed was selected. This transformant was cultured overnight in LB medium and a plasmid pTC2MetC24 was prepared using QIAprep8 Miniprep Kit (Qiagen). The base sequence of KiSS-1 peptide (having 24 amino acids at its C-terminal) structural gene portion of said plasmid was confirmed using an Applied Biosystems model 3100 DNA sequencer. The plasmid pTC2MetC24 was used to transform the E. coli MM294 (DE3), and thus KiSS-1 protein (having 24 amino acids at its C-terminal) expression strain, MM294 (DE3)/pTC2MetC24-1.3, was obtained.

### (e) Production of KiSS-1 peptide to which 24 amino acids are added at its C-terminal.

The MM294(DE3)/pTC2MetC24-1.3 was cultured with shaking in a 2-liter flask at 37°C for 8 hours using 1 L of LB medium (1% peptone, 0.5% yeast extract, 0.5% sodium chloride) containing 5.0 mg/L of tetracycline. The culture obtained was transferred to a 50-L fermenter charged with 19 L of a main fermentation medium (1.68% sodium monohydrogen phosphate, 0.3% potassium dihydrogen phosphate, 0.1% ammonium chloride, 0.05% sodium chloride, 0.025% magnesium sulfate, 0.02% antifoam, 0.00025% ferrous sulfate, 0.0005% thiamine hydrochloride, 1.5% glucose, 1.5% casamino acids), and cultivation under aeration and agitation was started at 30°C. When the turbidity of the culture arrived at about 500 Klett units, isopropyl-β-D-thiogalactopyranoside was added to a final concentration of 12 mg/L, and cultivation was carried out for further 6 hours. After completion of the cultivation, the culture fluid was centrifuged to give about 430 g of wet bacterial cells, which were frozen stored at -80°C.

### Example 2

To 70 g of the bacterial cells obtained in Example 1 was added 210 ml of a solution comprising 7 M guanidine hydrochloride, 100 mM Tris buffer and 1 mM EDTA (pH 8.0), and the mixture was dissolved under stirring and centrifuged (8,000 rpm, 60 minutes). The supernatant was diluted in a 4.2 L solution comprising 50 mM Tris buffer, 0.2 M arginine (pH 8.0), 1 mM reduced glutathione and 0.1 mM oxidized glutathione, and stood overnight in a low-temperature room. This solution was diluted 4 times with 1 M urea solution, adjusted to pH 6.0 with acetic acid, and applied to a SP-Toyopearl 550C column (5 cm ID × 20 cm L, Tosoh) equilibrated with 50 mM MES-NaOH buffer (pH 6.0), at a flow rate of 750 mL/hour, for adsorption. The column was washed with 50 mM MES-NaOH buffer + 0.2 M NaCl (pH 6.0) and then elution was carried out with 50 mM MES-NaOH buffer + 0.5 M NaCl (pH 6.0). This eluate was diluted 3 times with distilled water, was applied to a SP-5PW column (21.5 mm ID × 150 mm L, Tosoh) equilibrated with 50 mM MES-NaOH buffer (pH 6.0), at a flow rate of 5 mL/minute, for adsorption, and then stepwise gradient elution was carried out with 0 to 80% B (B = 1 M NaCl + 50 mM MES-NaOH buffer, pH 6.0) to pool KiSS-1-24 amino acid adduct fraction. This eluted fraction was applied to a C4P-50 column (21.5 mm ID × 300 mm L, Showa Denko) equilibrated with 0.1% trifluoroacetic acid, for adsorption, and stepwise gradient elution was carried out with 20 to 70% B (B = 0.1% trifluoroacetic acid + 80% acetonitrile) at a flow rate of 5 mL/minute to give KiSS-1-24 amino acid adduct fraction. This fraction obtained was lyophilized to give a lyophilizate powder of KiSS-1-24 amino acid adduct fraction. The lyophilized powder was dissolved in a solution comprising 0.1 M acetic acid and 6 M urea. Then, about 1.5 mg of DMAP-CN (1-cyano-4-dimethylaminopyridinium tetrafluoroborate) was added this solution, and the reaction was allowed to proceed at room temperature for 15 minutes. After completion of the reaction, the reaction mixture was applied to a Sephadex G-25 column (2.5 cm ID × 50 cm L, Pharmacia) equilibrated with 50 mM monopotassium phosphate and development was effected using the same 50 mM monopotassium phosphate as used for equilibration at a flow rate of 10 ml/minute to give a fraction containing the S-cyanylated KiSS-1 peptide-24 amino acid adduct protein. This eluate was concentrated and desalted using Centriplus (fractional molecular weight of 3kDa:Millipore) to give a desalted solution of the KiSS-1-24 amino acid adduct. Urea was added to this desalted solution to a final concentration of 6 M, 25% aqueous ammonia was further added to an ammonia concentration of 3 M, and the reaction was allowed to proceed at room temperature for 15 minutes. After completing the reaction, the reaction mixture was adjusted to pH 6.0 with acetic acid to give KiSS-1 peptide. This reaction mixture was applied to a Sephadex G-25 column (2.5 cm ID × 50 cm L) equilibrated with 50 mM monopotassium phosphate and development was effected using the same 50 mM monopotassium phosphate as used for equilibration at a flow rate of 10 ml/minute to give KiSS-1 peptide fraction. This fraction was further applied to a C4P-50 column (21.5 mm ID × 300 mm L, Showa Denko) equilibrated with 0.1% trifluoroacetic acid, for adsorption. After washing the column, stepwise gradient elution was carried out with 20 to 60% B (B: 80% acetonitrile/0.1% trifluoroacetic acid) at a flow rate of 5 mL/minute. The KiSS-1 peptide fractions obtained were pooled and lyophilized to give about 0.35 mg lyophilizate powder of KiSS-1 peptide.

### Example 3

### Determination of KiSS-1 Peptide Characteristics

### a) Amino Acid Composition Analysis

The amino acid composition was determined using an amino acid analyzer (Hitachi model L-8500A amino acid analyzer). As a result, the amino acid composition was in agreement with that deduced from the base sequence of the DNA for KiSS-1 peptide (Table 1).

**TABLE 1**

| Amino acid | Number of residues per mole | Value deduced from base sequence of KiSS-1 peptide |
|---|---|---|
| Asx | 3.4 | 4 |
| Thr*) | 0.9 | 1 |
| Ser*) | 7.1 | 8 |
| Glx | 7.1 | 7 |
| Pro | 8.2 | 8 |
| Gly | 5.1 | 5 |
| Ala | 3.0 | 3 |
| Cys | 0 | 0 |
| Val | 1.9 | 2 |
| Met | 0 | 0 |
| Ile | 1.0 | 1 |
| Leu | 5 | 5 |
| Tyr | 1.0 | 1 |
| Phe | 1.9 | 2 |
| His | 1.0 | 1 |
| Lys | 1.0 | 1 |
| Arg | 3.9 | 4 |
| Trp | 0.4 | 1 |
| Acid hydrolysis (mean value after acid hydrolysis in 6 N HCl-4% thioglycolic acid for 24-48 hrs) | | |

| | | |
|---|---|---|
| *) Value obtained by extrapolation to 0 hr. b) N-terminal Amino Acid Sequence Analysis | | |

The N-terminal amino acid sequence was determined using a gaseous phase protein sequencer (PE Applied Biosystems model 492). As a result, the N-terminal amino acid sequence was in agreement with that deduced from the base sequence of the DNA for KiSS-1 peptide (Table 2).

**TABLE 2**

| N-terminal Amino Acid Sequence Analysis | | |
|---|---|---|
| Residue No. | PTH*)-amino acid Detected | Amino acid deduced from base sequence of KiSS-1 peptide |
| 1 | Gly (56) | Gly |
| 2 | Thr (52) | Thr |
| 3 | Ser (41) | Ser |
| 4 | Leu (45) | Leu |
| 5 | Ser (33) | Ser |
| 6 | Pro (28) | Pro |
| 7 | Pro (34) | Pro |
| 8 | Pro (30) | Pro |
| 9 | Glu (14) | Glu |
| 10 | Ser (12) | Ser |
| Analysis was made using 100 pmol. | | |

| | | |
|---|---|---|
| *) Phenylthiohydantoin. | | |

### Example 4

### Biological Activity Assay

The activity assay of the KiSS-1 peptide obtained in Example 2 was performed by the method described in Example 3 of WO 99/33976 (intracellular Ca ion concentration-increasing activity) and it was confirmed that its activity was equivalent to that of a sample purified from human placenta extract.

### Example 5

### Production of DNA encoding a KiSS-1 Peptide to which 24 amino acids are added at its C-terminal (2)

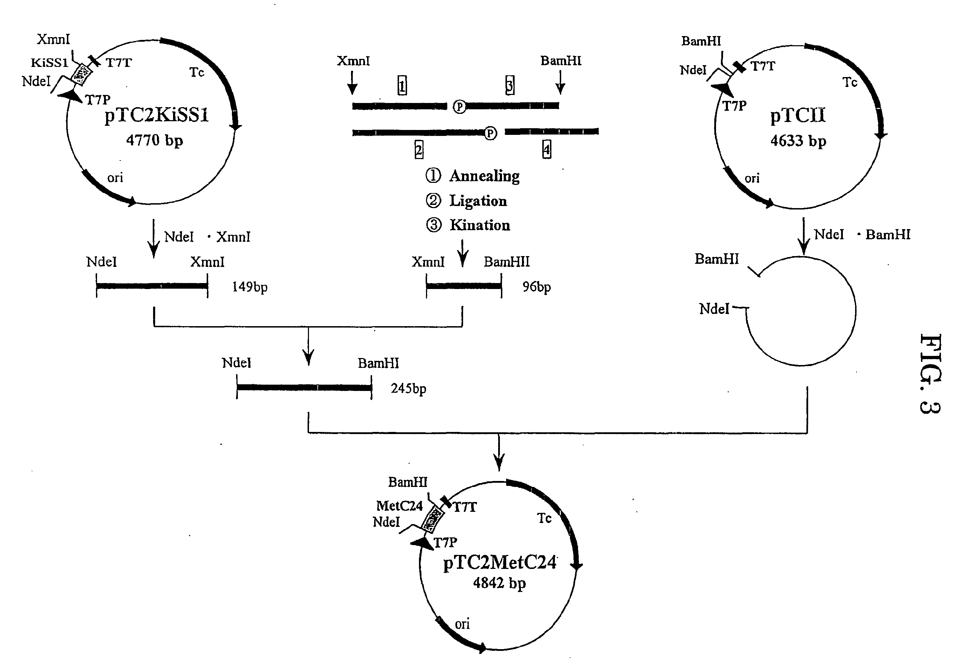

pTC2MetC24-1.3, an expression vector for KiSS-1 peptide to which 24 amino acids are added at its C-terminal, was constructed using pTC2KiSS1 in place of pTFC-KiSS-1, the expression vector for KiSS-1 Peptide used in Example 1(c) (Fig.3).

### Industrial Applicability

According to the method of the present invention, it is possible to industrially mass-produce KiSS-1 Peptide or a salt thereof, which can be used as a prophylactic or therapeutic drug for cancers (for example, lung cancer, stomach cancer, liver cancer, pancreatic cancer, colorectal cancer, rectal cancer, colon cancer, prostate cancer, ovarian cancer, uterine cancer, or breast cancer, etc.) as well as for choriocarcinoma, vesicular mole, invasive mole, miscarriage, fetal maldevelopment, saccharometabolic disorder, lipid metabolic disorder, or induction of delivery.

## Claims

1. A method for producing a KiSS-1 peptide or a salt thereof, which comprises subjecting a fused protein, a peptide or a salt thereof in which a KiSS-1 peptide is ligated to the N-terminal of a low-molecular peptide having cysteine at its N-terminal to the cleavage reaction of the peptide linkage on the amino group side of said cysteine residue.

2. A method for producing a KiSS-1 peptide or a salt thereof, which comprises cultivating a transformant having a vector comprising DNA encoding a fused protein or peptide in which a KiSS-1 peptide is ligated to the N-terminal of a low-molecular peptide having cysteine at its N-terminal to express the fused protein, the peptide or a salt thereof; and subjecting the expressed fused protein, the peptide or the salt thereof to the cleavage reaction of the peptide linkage on the amino group side of said cysteine residue.

3. The method according to Claim 1 or 2, wherein the C-terminal of the KiSS-1 peptide is an amide.

4. The method according to Claim 1 or 2, wherein the cleavage reaction is an S-cyanylation reaction followed by an ammonolysis or a hydrolysis.

5. The method according to Claim 1 or 2, wherein the KiSS-1 peptide is a peptide comprising an amino acid sequence of SEQ ID NO: 1.

6. The method according to Claim 1 or 2, wherein the KiSS-1 peptide is (i) a peptide having an amino acid sequence of position 40 to position 54 from the N-terminal in the amino acid sequence of SEQ ID NO: 1; (ii) a peptide having an amino acid sequence of position 45 to position 54 from the N-terminal in the amino acid sequence of SEQ ID NO: 1; (iii) a peptide having an amino acid sequence of position 46 to position 54 from the N-terminal in the amino acid sequence of SEQ ID NO: 1; or (iv) a peptide having an amino acid sequence of position 47 to position 54 from the N-terminal in the amino acid sequence of SEQ ID NO: 1.

7. The method according to Claim 1 or 2, wherein the low-molecular peptide having cysteine at the N-terminal is a peptide consisting of about 10 to about 50 amino acid residues.

8. The method according to Claim 1 or 2, wherein the low-molecular peptide is a partial peptide on the C-terminal side of a precursor protein comprising the KiSS-1 peptide, and has an amino acid sequence starting from an amino acid residue adjacent to the C-terminal amino acid of the KiSS-1 peptide.

9. The method according to Claim 1 or 2, wherein the low- molecular peptide having cysteine at the N-terminal is a peptide that comprises the amino acid sequence of SEQ ID NO: 3 and has cysteine residue at the N-terminal.

10. The method according to Claim 1 or 2, wherein the low-molecular peptide having cysteine at the N-terminal is a peptide that comprises an amino acid sequence of SEQ ID NO: 3 and has cysteine residue at the N-terminal; the KiSS-1 peptide is a peptide having an amino acid sequence of SEQ ID NO: 1; and the KiSS-1 peptide to be produced is a peptide having an amino acid sequence of SEQ ID NO: 1 with an amide form of the C-terminal.

11. A fused protein, a peptide or a salt thereof, in which a KiSS-1 peptide is ligated to the N-terminal of a low-molecular peptide having cysteine at its N-terminal.

12. The fused protein, the peptide or the salt thereof according to Claim 11, comprising an amino acid sequence of SEQ ID NO: 5.

13. DNA comprising DNA encoding the fused protein or the peptide according to Claim 11.

14. DNA according to Claim 13, having (i) a base sequence of SEQ ID NO: 6; or (ii) a base sequence of SEQ ID NO: 7.

15. A vector comprising the DNA according to Claim 13.

16. A transformant having the vector according to Claim 15.

17. Escherichia coli MM294 (DE3)/pTC2MetC24-1.3 identified as FERM BP-7823.


[SEQUENCE LISTING]

<110> Takeda Chemical Industries, Ltd.

<120> Process for Producing KiSS-1 peptide

<130> P04-501PCT

<150> JP 2002-005180
<151> 2002-01-11

<160> 21

<210> 1
<211> 54
<212> PRT
<213> Homo sapiens

<220>
<223> the C-terminus of the polypeptide is amide (-CONH2) form

<400> 1
Gly Thr Ser Leu Ser Pro Pro Pro Glu Ser Ser Gly Ser Arg Gln Gln
1               5                   10                  15
Pro Gly Leu Ser Ala Pro His Ser Arg Gln Ile Pro Ala Pro Gln Gly
            20                  25                  30
Ala Val Leu Val Gln Arg Glu Lys Asp Leu Pro Asn Tyr Asn Trp Asn
        35                  40                  45
Ser Phe Gly Leu Arg Phe
    50              54

<210> 2
<211> 162
<212> DNA
<213> Homo sapiens

<400> 2
ggtacttctc tgtctccgcc gccggaatct tctggttctc gtcagcagcc gggtctgtct    60
gctccgcact ctcgtcagat cccggctccg cagggtgctg ttctggttca gcgtgaaaaa   120
gacctgccga actacaactg gaactctttc ggtctgcgtt tc                      162

<210> 3
<211> 24
<212> PRT
<213> Homo sapiens

<400> 3
Gly Lys Arg Glu Ala Ala Pro Gly Asn His Gly Arg Ser Ala Gly Arg
1               5                   10                  15

Gly Trp Gly Ala Gly Ala Gly Gln
            20                  24

<210> 4
<211> 72
<212> DNA
<213> Homo sapiens

<400> 4
ggtaaacgtg aagctgctcc gggtaaccac ggtcgttctg ctggtcgtgg ttggggtgct    60
ggtgctggtc ag                                                         72

<210> 5
<211> 79
<212> PRT
<213> Homo sapiens

<400> 5

Gly Thr Ser Leu Ser Pro Pro Pro Glu Ser Ser Gly Ser Arg Gln Gln
1               5                   10                  15
Pro Gly Leu Ser Ala Pro His Ser Arg Gln Ile Pro Ala Pro Gln Gly
            20                  25                  30
Ala Val Leu Val Gln Arg Glu Lys Asp Leu Pro Asn Tyr Asn Trp Asn
        35                  40                  45
Ser Phe Gly Leu Arg Phe Cys Gly Lys Arg Glu Ala Ala Pro Gly Asn
    50                  55                  60
His Gly Arg Ser Ala Gly Arg Gly Trp Gly Ala Gly Ala Gly Gln
65                  70                  75

<210> 6
<211> 237
<212> DNA
<213> Homo sapiens

<400> 6
ggtacttctc tgtctccgcc gccggaatct tctggttctc gtcagcagcc gggtctgtct      60
gctccgcact ctcgtcagat cccggctccg cagggtgctg ttctggttca gcgtgaaaaa     120
gacctgccga actacaactg gaactctttc ggtctgcgtt tctgcggtaa acgtgaagct     180
gctccgggta accacggtcg ttctgctggt cgtggttggg gtgctggtgc tggtcag        237

<210> 7
<211> 237
<212> DNA
<213> Homo sapiens

<400> 7
ggtacttctc tgtctccgcc gccggaatct tctggttctc gtcagcagcc gggtctgtct      60
gctccgcact ctcgtcagat cccggctccg cagggtgctg ttctggttca gcgtgaaaaa     120
gacctgccga actacaactg gaactctttc ggtctgcgtt tctgtggtaa acgtgaagct     180
gctccgggta accacggtcg ttctgctggt cgtggttggg gtgctggtgc tggtcag        237

<210> 8
<211> 42
<212> DNA
<213> Artificial Sequence

<220>
<223> Primer

<400> 8
ctttcggtct gcgtttctgc ggtaaacgtg aagctgctcc gg                        42

<210> 9
<211> 47
<212> DNA
<213> Artificial Sequence

<220>
<223> Primer

<400> 9
gttacccgga gcagcttcac gtttaccgca gaaacgcaga ccgaaag                   47

<210> 10
<211> 54
<212> DNA
<213> Artificial Sequence

<220>
<223> Primer

<400> 10
gtaaccacgg tcgttctgct ggtcgtggtt ggggtgctgg tgctggtcag tgag   54

<210> 11
<211> 53

<212> DNA
<213> Artificial Sequence

<220>
<223> Primer

<400> 11
gatcctcact gaccagcacc agcaccccaa ccacgaccag cagaacgacc gtg    53

<210> 12
<211> 96
<212> DNA
<213> Homo sapiens

<400> 12
ctttcggtct gcgtttctgc ggtaaacgtg aagctgctcc gggtaaccac ggtcgttctg    60
ctggtcgtgg ttggggtgct ggtgctggtc agtgag                              96

<210> 13
<211> 149
<212> DNA
<213> Homo sapiens

<400> 13
tatgggtact tctctgtctc cgccgccgga atcttctggt tctcgtcagc agccgggtct    60
gtctgctccg cactctcgtc agatcccggc tccgcagggt gctgttctgg ttcagcgtga   120
aaaagacctg ccgaactaca actggaact                                     149

<210> 14
<211> 245
<212> DNA
<213> Homo sapiens

<400> 14
tatgggtact tctctgtctc cgccgccgga atcttctggt tctcgtcagc agccgggtct    60
gtctgctccg cactctcgtc agatcccggc tccgcagggt gctgttctgg ttcagcgtga   120
aaaagacctg ccgaactaca actggaactc tttcggtctg cgtttctgcg gtaaacgtga   180
agctgctccg ggtaaccacg gtcgttctgc tggtcgtggt tggggtgctg gtgctggtca   240
gtgag                                                               245

<210> 15
<211> 145
<212> PRT
<213> Homo sapiens

<400> 15

<210> 16

<211> 152
<212> PRT
<213> Mus musculus

<400> 16
Met Tyr Leu Arg Phe Gly Val Asp Val Cys Ser Leu Ser Pro Trp Lys
                5                   10                  15
Glu Thr Val Asp Leu Pro Leu Pro Pro Arg Met Ile Ser Met Ala Ser
            20                  25                  30
Trp Gln Leu Leu Leu Leu Leu Cys Val Ala Thr Tyr Gly Glu Pro Leu
        35                  40                  45
Ala Lys Val Ala Pro Gly Ser Thr Gly Gln Gln Ser Gly Pro Gln Glu
    50                  55                  60
Leu Val Asn Ala Trp Glu Lys Glu Ser Arg Tyr Ala Glu Ser Lys Pro
65                  70                  75                  80
Gly Ser Ala Gly Leu Arg Ala Arg Arg Ser Ser Pro Cys Pro Pro Val
                85                  90                  95
Glu Gly Pro Ala Gly Arg Gln Arg Pro Leu Cys Ala Ser Arg Ser Arg
            100                 105                 110
Leu Ile Pro Ala Pro Arg Gly Ala Val Leu Val Gln Arg Glu Lys Asp
        115                 120                 125
Leu Ser Thr Tyr Asn Trp Asn Ser Phe Gly Leu Arg Tyr Gly Arg Arg
    130                 135                 140
Gln Ala Ala Arg Ala Ala Arg Gly
145                 150

<210> 17
<211> 156
<212> PRT
<213> Mus musculus

<400> 17
Met Tyr Leu Arg Phe Gly Val Asp Val Cys Ser Leu Ser Pro Trp Lys
                5                   10                  15
Glu Thr Val Asp Leu Pro Leu Pro Pro Arg Met Ile Ser Met Ala Ser
            20                  25                  30
Trp Gln Leu Leu Leu Leu Leu Cys Val Ala Thr Tyr Gly Glu Pro Leu
        35                  40                  45
Ala Lys Val Ala Pro Leu Val Lys Pro Gly Ser Thr Gly Gln Gln Ser
    50                  55                  60
Gly Pro Gln Glu Leu Val Asn Ala Trp Glu Lys Glu Ser Arg Tyr Ala
65                  70                  75                  80
Glu Ser Lys Pro Gly Ser Ala Gly Leu Arg Ala Arg Arg Ser Ser Pro
                85                  90                  95
Cys Pro Pro Val Glu Gly Pro Ala Gly Arg Gln Arg Pro Leu Cys Ala
            100                 105                 110
Ser Arg Ser Arg Leu Ile Pro Ala Pro Arg Gly Ala Val Leu Val Gln
        115                 120                 125
Arg Glu Lys Asp Leu Ser Thr Tyr Asn Trp Asn Ser Phe Gly Leu Arg
    130                 135                 140
Tyr Gly Arg Arg Gln Ala Ala Arg Ala Ala Arg Gly
145                 150                 155

<210> 18
<211> 130
<212> PRT
<213> Rattus norvegicus

<400> 18
Met Thr Ser Leu Ala Ser Trp Gln Leu Leu Leu Leu Leu Cys Val Ala
                5                   10                  15
Ser Phe Gly Glu Pro Leu Ala Lys Met Ala Pro Val Val Asn Pro Glu
            20                  25                  30
Pro Thr Gly Gln Gln Ser Gly Pro Gln Glu Leu Val Asn Ala Trp Gln
        35                  40                  45
Lys Gly Pro Arg Tyr Ala Glu Ser Lys Pro Gly Ala Ala Gly Leu Arg
    50                  55                  60
Ala Arg Arg Thr Ser Pro Cys Pro Pro Val Glu Asn Pro Thr Gly His

65                  70                  75                  80
Gln Arg Pro Pro Cys Ala Thr Arg Ser Arg Leu Ile Pro Ala Pro Arg
                85                  90                  95
Gly Ser Val Leu Val Gln Arg Glu Lys Asp Met Ser Ala Tyr Asn Trp
            100                 105                 110
Asn Ser Phe Gly Leu Arg Tyr Gly Arg Arg Gln Val Ala Arg Ala Ala
        115                 120                 125
Arg Gly
    130

<210> 19
<211> 449
<212> DNA
<213> Mus musculus

<400> 19
atgtatctga gatttggcgt tgatgtctgc agcctgagtc cctggaagga gactgtagac  60
ctgccccttc ctcccagaat tctcaatggc ttcttggcag ctgctgcttc tcctctgtgt 120
cgccacctat ggggagccgc tggcaaaagt gaagcctgga cacaggccag cagtccggac 180
cccaggaact cgttaatgcc tgggaaaagg aatcgcggta tgcagagagc aagcctgggt 240
gcagggctgc gcgctcgtag gtcgtcgcca tgcccgccgg ttgagggccc cgcggggcgc 300
cagcggcccc tgtgtgcctc gcagtcgcct gatccctgcg ccccgcggag cggtgctggt 360
gcagcgggag aaggacctgt ccacctacaa ctggaactcc cggcctgcgc tacggcagga 420
ggcaggcggc gcgggcagca cggggctga                                   449

<210> 20
<211> 458
<212> DNA
<213> Mus musculus

<400> 20
atgtatctga gatttggcgt tgatgtctgc agcctgagtc cctggaagga gactgtagac  60
ctgccccttc ctcccagaat tctcaatggc ttcttggcag ctgctgcttc tcctctgtgt 120
cgccacctat ggggagccgc tggcaaaagt ggcacctttg gaagcctgga tccacaggcc 180
agcagtccgg accccaggaa ctcgttaatg cctgggaaaa ggaatcgcgg tatgcagaga 240
aagcctgggt ctgcagggct gcgcgctcgt aggtcgtcgc catgcccgcc ggttgagggc 300
cccgcggggc gccagcggcc tgtgtgcctc ccgcagtcgc ctgatccctg cgccccgcgg 360
agcggtgctg gtgcagcggg agaaggacct gtcgacctac ctggaactcc ttcggcctgc 420
gctacggcag gaggcaggcg gcgcgggcag cacggggc                         458

<210> 21
<211> 390
<212> DNA
<213> Rattus norvegicus

<400> 21
atgacctcgc tggcttcttg gcagctgctg cttctcctct gtgtggcctc ttttggggag  60
ccactggcaa aaatggcacc tgtggtgaac cctgaaccca caggccaaca gtccggaccc 120
caggaactcg ttaatgcctg gcaaaagggc ccgcggtatg cagagagcaa gcctggggct 180
gcaggactgc gcgctcgccg aacatcgcca tgcccgccgg tggagaaccc cacggggcac 240
cagcggcccc cgtgtgccac ccgcagtcgc ctgatccctg cgccccgcgg atcggtgctg 300
gtgcagcgcg agaaggacat gtcagcctac aactggaact cctttggcct gcgctacggc 360
aggaggcagg tggcgcgggc ggcacggggc                                   390